# EUROPÄISCHE PATENTSCHRIFT

(11) **EP 0 810 975 B1**
(45) Veröffentlichungstag und Bekanntmachung des Hinweises auf die Patenterteilung: **12.05.1999**
(21) Anmeldenummer: 96904851.1
(22) Anmeldetag: 22.02.1996
(51) Int. Cl.: C01B 33/158

(54) **VERFAHREN ZUR HERSTELLUNG HYDROPHILER ODER TEILWEISE HYDROPHILER, ANORGANISCHER AEROGELE**
METHOD OF PRODUCING HYDROPHILIC OR PARTIALLY HYDROPHILIC INORGANIC AEROGELS
PROCEDE DE PRODUCTION D'AEROGELS INORGANIQUES HYDROPHILES OU PARTIELLEMENT HYDROPHILES

(30) Priorität: 22.02.1995 DE 19506141
(43) Veröffentlichungstag der Anmeldung: 10.12.1997
(73) Patentinhaber: CABOT CORPORATION, Boston, MA 02109 (US)
(72) Erfinder: SCHWERTFEGER, Fritz, D-60529 Frankfurt am Main (DE)
(74) Vertreter: Luderschmidt, Schüler & Partner GbR
(86) Internationale Anmeldenummer: EP9600741
(87) Internationale Veröffentlichungsnummer: WO9626890

(56) Entgegenhaltungen:
- EP-A- 0 018 955
- EP-A- 0 537 851
- EP-A- 0 658 513
- WO-A-92/03378
- WO-A-95/06617

## Beschreibung

Die Erfindung betrifft ein Verfahren zur Herstellung hydrophiler oder teilweise hydrophiler, anorganischer Aerogele aus durch Silylierung modifizierten hydrophoben, anorganischen Aerogelen.

Aerogele, insbesondere solche mit Porositäten über 60 % und Dichten unter 0,6 g/cm³, weisen eine äußerst geringe thermische Leitfähigkeit auf und finden deshalb Anwendung als Wärmeisolationsmaterial wie z.B. in der EP-A-0 171 722 beschrieben.

Aerogele im weiteren Sinn, d.h. im Sinne von "Gel mit Luft als Dispersionsmittel", werden durch Trocknung eines geeigneten Gels hergestellt. Unter den Begriff "Aerogel" in diesem Sinne, fallen Aerogele im engeren Sinn, Xerogele und Kryogele. Dabei wird ein getrocknetes Gel als Aerogel im engeren Sinn bezeichnet, wenn die Flüssigkeit des Gels bei Temperaturen oberhalb der kritischen Temperatur und ausgehend von Drücken oberhalb des kritischen Druckes weitestgehend entfernt wird. Wird die Flüssigkeit des Gels dagegen unterkritisch, beispielsweise unter Bildung einer Flüssig-Dampf-Grenzphase entfernt, dann bezeichnet man das entstandene Gel als Xerogel.

Bei der Verwendung des Begriffs Aerogele in der vorliegenden Anmeldung handelt es sich um Aerogele im weiteren Sinn, d.h. im Sinn von "Gel mit Luft als Dispersionsmittel".

Nicht unter diesen Begriff fallen aus der älteren Literatur bekannte Xerogele, die z.B. durch Fällung von Kieselsäure (z. B. DE-A-30 25 437, DD-A-296 898) erhalten werden, oder als pyrogene Kieselsäure (z.B. Aerosil®), anfallen. In diesen Fällen bildet sich jedoch während der Herstellung kein über größere Distanzen homogenes dreidimensionales Gelnetzwerk aus.

Bei Aerogelen kann man grundsätzlich zwischen anorganischen und organischen Aerogelen unterschieden.
Anorganische Aerogele sind schon seit 1931 bekannt (S.S.Kistler, Nature 1931,127,741). Seitdem sind aus unterschiedlichsten Ausgangsmaterialien Aerogele dargestellt worden. Dabei konnten z. B. SiO₂-, Al₂O₃-, TiO₂-, ZrO₂-, SnO₂-, Li₂O-, CeO₂-, V₂O₅-Aerogele und Mischungen aus diesen hergestellt werden (H.D. Gesser, P.C.Goswami, Chem.Rev.1989, 89,765ff).
Seit einigen Jahren sind auch organische Aerogele aus unterschiedlichsten Ausgangsmaterialien, z.B. aus Melaminformaldehyd, bekannt (R.W. Pekala, J. Mater. Sci. 1989, 24, 3221).

Anorganische Aerogele können dabei auf zwei prinzipiell unterschiedlichen Wegen hergestellt werde.

Zum einen können SiO₂-Aerogele beispielsweise durch saure Hydrolyse und Kondensation von Tetraethylorthosilikat in Ethanol hergestellt werden. Dabei entsteht ein Gel, das durch überkritische Trocknung unter Erhaltung der Struktur getrocknet werden kann. Auf dieser Trocknungstechnik basierende Herstellungsverfahren sind z.B. aus der EP-A-0 396 076 , der WO 92/03378 und der WO 95/06617 bekannt.

Eine prinzipielle Alternative zur überkritischen Trocknung bietet ein Verfahren zur unterkritischen Trocknung von SiO₂-Gelen. Das SiO₂-Gel kann dabei beispielsweise durch saure Hydrolyse von Tetraalkoxysilanen in einem geeigneten organischen Lösungsmittel mittels Wasser erhalten werden. Nach Austausch des Lösungsmittels gegen ein geeignetes organisches Lösungsmittel wird in einem weiteren Schritt das erhaltene Gel mit einem Silylierungsmittel umgesetzt. Das dabei entstehende SiO₂-Gel kann anschließend aus einem organischen Lösungsmittel heraus an der Luft getrocknet werden. Damit können Aerogele mit Dichten unter 0,4 g/cm³ und Porositäten über 60 % erreicht werden. Das auf dieser Trocknungstechnik basierende Herstellungsverfahren ist ausführlich in der WO 94/25149 beschrieben.

Die oben beschriebenen Gele können darüber hinaus vor der Trocknung in der alkohol-wäßrigen Lösung mit Tetraalkoxysilanen versetzt und gealtert werden, um die Gelnetzwerkstärke zu erhöhen, wie z.B. in der WO 92/20623 offenbart.

Die bei den oben beschriebenen Verfahren als Ausgangsmaterialien verwendeten Tetraalkoxysilane stellen jedoch einen außerordentlich hohen Kostenfaktor dar.

Eine nicht unerhebliche Kostensenkung kann durch die Verwendung von Wasserglas als Ausgangsmaterial für die Herstellung der SiO₂-Gele erreicht werden. Dazu kann beispielsweise aus einer wäßrigen Wasserglaslösung mit Hilfe eines lonenaustauscherharzes eine Kieselsäure hergestellt werden, die durch Zugabe einer Base zu einem SiO₂-Gel polykondensiert. Nach Austausch des wäßrigen Mediums gegen ein geeignetes organisches Lösungsmittel wird dann in einem weiteren Schritt das erhaltene Gel mit einem chlorhaltigen Silylierungsmittel umgesetzt. Das dabei entstehende, auf der Oberfläche z. B. mit Methylsilylgruppen modifizierte SiO₂-Gel kann anschließend ebenfalls aus einem organischen Lösungsmittel heraus an der Luft getrocknet werden. Das auf dieser Technik basierende Herstellungsverfahren ist aus der DE-A-43 42 548 bekannt.

Verfahrensalternativen bezüglich der Herstellung eines SiO₂-Hydrogels auf der Basis von Wasserglas mit anschließender unterkritischer Trocknung werden in den deutschen Patentanmeldungen 195 41 715.1 und 195 41 992.8 beschrieben.

In der deutschen Patentanmeldung 195 02 453.2 wird darüber hinaus die Verwendung von chlorfreien Silylierungsmitteln bei der Herstellung von unterkritisch getrockneten Aerogelen beschrieben.

In der deutschen Patentanmeldung 195 34 198.8 wird ferner eine Organofunktionalisierung mittels organofunktionalisierter Silylierungsmittel bei der Herstellung von unterkritisch getrockneten Aerogelen beschrieben.

Ferner wird in der EP-A-0 606 638 die Herstellung von Kohlenstoffpartikel enthaltender SiO₂-Aerogele durch Erhitzen organisch modifizierter SiO₂-Aerogele in Gegenwart mindestens eines pyrolysierbaren Kohlenwasserstoffgases und/oder mindestens eines inerten Gases offenbart. Dabei werden die organischen Gruppen auf der Oberfläche der Aerogele bzw. des Kohlenwasserstoffgases in einer sauerstoffreien Atmosphäre zu elementarem Kohlenstoff oxidiert.

Die durch überkritische Trocknung erhaltenen Aerogele sind, je nach dem bei der überkritischen Trocknung verwendeten Lösungsmittel, aufgrund von OH Gruppen auf der inneren Oberfläche hydrophil (überkritische Trocknung aus CO₂) oder aufgrund von Alkoxygruppen auf der inneren Oberfläche kurzfristig hydrophob (überkritische Trocknung aus Alkoholen). Langfristig erfolgt jedoch eine Reaktion der Alkoxygruppen mit Wasser aus der Umgebung, was zur Bildung von Hydroxidgruppen auf der inneren Oberfläche führt. Daraus resultiert wieder ein hydrophiles Aerogel.

Die Ausbildung eines hydrophilen Aerogels kann durch einen Hydrophobisierungsschritt während der überkritischen Trocknung vermieden werden. Dazu kann ein Hydrophobisierungsmittel wie z.B. Me₂Si(OMe)₂ eingesetzt werden, z.B. in der EP-A-0 396 076 offenbart. Die dabei erhaltenen Aerogele sind aufgrund der Methylsilylgruppen auf der inneren Oberfläche dauerhaft hydrophob.

Unterkritisch getrocknete Aerogele sind bedingt durch das Herstellungsverfahren (Silylierung vor der Trocknung) dauerhaft hydrophob. Da die unterkritische Trocknung von Aerogelen unreaktive, hydrophobe innere Geloberflächen voraussetzt, ist eine direkte Herstellung von hydrophilen, unterkritisch getrockneten Aerogelen nicht möglich.

Für viele Anwendungen ist jedoch gerade eine hydrophile oder teilweise hydrophile innere Oberfläche der Aerogele notwendig oder von Vorteil.

Aufgabe der vorliegenden Erfindung war es daher, ein Verfahren zur Herstellung hydrophiler oder teilweise hydrophiler, anorganischer Aerogele bereitzustellen, ohne jedoch den Nachteil der überkritischen Trocknung, die einen unverhältnismäßig hohen verfahrenstechnischen Aufwand darstellt, in Kauf nehmen zu müssen.

Diese Aufgabe wird gelöst durch ein Verfahren, bei dem man ein durch Silylierung modifiziertes hydrophobes, anorganisches, vorzugsweise unterkritisch getrocknetes Aerogel, in Gegenwart von Sauerstoff bei Temperaturen im Bereich von 100 bis 1000°C pyrolysiert.

Gegenwart von Sauerstoff bedeutet dabei in der vorliegenden Anmeldung, daß eine zur Oxidation der Aerogel-Oberflächengruppen ausreichende Menge Sauerstoff vorhanden ist, wobei diese Menge in jeder dem Fachmann bekannten Zusammensetzung vorliegen kann (z.B.: Luft, Sauerstoff-Luft-Gemisch und/oder Sauerstoff-lnertgas-Gemisch).

Unter einem anorganischen Aerogel ist in der vorliegenden Anmeldung ein Aerogel zu verstehen, das auf der Basis von anorganischen Materialien hergestellt wurde, und das durch Silylierung modifiziert wurde.

Bevorzugt sind Aerogele mit hydrophoben Oberflächengruppen überwiegend aus SiO₂, Al₂O₃, TiO₂, ZrO₂ oder Mischungen davon. Die Herstellung von Aerogelen mit hydrophoben Oberflächengruppen kann dabei nach allen dem Fachmann bekannten Verfahren durchgeführt werden. Besonders bevorzugt sind hydrophobe SiO₂-haltige Aerogele, insbesondere SiO₂-Aerogele.

In einer bevorzugten Ausführungsform wird das Aerogel bei Temperaturen von 100 bis 1000°C an Luft pyrolysiert, besonders bevorzugt zwischen 150 und 800°C in einem leichten Luftstrom, insbesondere zwischen 250 und 650°C.

Die Pyrolysezeit wird im wesentlichen durch die Oberflächenmodifikation und die Materialdichte der Aerogele bestimmt. Vorzugsweise liegt die Pyrolysezeit unter 10 Stunden, besonders bevorzugt unter 1 Stunde.

Will man teilweise hydrophile Aerogele herstellen, so ist die Temperatur und die Pyrolysezeit so einzustellen, das nur ein entsprechender Teil der organischen Oberflächengruppen pyrolysiert.

Tragen die Aerogele auf ihrer inneren Oberfläche unterschiedliche organische Gruppen, so kann die unterschiedliche Zersetzungstemperatur der einzelnen organischen Gruppen dazu genutzt werden, die entsprechenden Gruppen kontrolliert und entsprechend des einzustellenden Hydrophiliegrades zu pyrolysieren. Bei Aerogelen, die sowohl Phenyl- als auch Methylgruppen auf ihrer inneren Oberfläche aufweisen, können dann z. B. teilweise hydrophile Eigenschaften dadurch erreicht werden, daß durch Pyrolyse bei 450°C zwar die Methylgruppen auf der Oberfläche eines entsprechenden Aerogels gegen Hydroxidgruppen ausgetauscht werden, nicht jedoch vorhandene Phenylgruppen.

Die erfindungsgemäße Darstellung der hydrophilen Aerogele wird im folgenden anhand von Ausführungsbeispielen näher beschrieben, ohne dadurch beschränkt zu werden.

### Beispiel 1

1 1 einer Natriumwasserglaslösung (mit einem Gehalt von 7 Gew.-% SiO₂ und einem Na₂O:SiO₂ Verhältnis von 1:3,3) wurde zusammen mit 0,5 1 eines sauren lonenaustauscherharzes (Styroldivinylbenzolcopolymer mit Sulfonsäuregruppen, handelsüblich unter dem Namen ®Duolite C20) gerührt, bis der pH-Wert der wäßrigen Lösung 2,3 war. Anschließend wurde das lonenaustauscherharz abfiltriert und die wäßrige Lösung mit 1 molarer NaOH-Lösung auf einen pH-Wert von 4,7 eingestellt. Danach wurde das entstandene Gel noch 3 Stunden bei 85°C gealtert und anschließend das Wasser mit 3 1 Aceton gegen Aceton ausgetauscht. Anschließend wurde das acetonhaltige Gel mit Trimethylchlorsilan silyliert (5 Gew.-% Trimethylchlorsilan pro Gramm nasses Gel). Die Trocknung des Gels erfolgte an Luft (3 Stunden bei 40°C, dann 2 Stunden bei 50°C und 12 Stunden bei 150°C).

Das so erhaltene, transparente Aerogel hatte eine Dichte von 0,2 g/cm³, seine spezifische Oberfläche nach BET lag bei 480 m²/g und es war dauerhaft hydrophob.

Das so hergestellte, dauerhaft hydrophobe Aerogel wurde bei 600°C in einem leichten Luftstrom mittels einem Röhrenofen 1 Stunde pyrolysiert. Das erhaltene transparente Aerogel hatte eine Dichte von 0,21 g/cm³, eine spezifische Oberfläche nach BET von 450 m²/g, eine Wärmeleitfähigkeit λ von 20 mW/mK und war hydrophil.

### Beispiel 2

1 1 einer Natriumwasserglaslösung (mit einem Gehalt von 7 Gew.-% SiO₂ und einem Na₂O:SiO₂ Verhältnis von 1:3,3) wurde zusammen mit 0,5 1 eines sauren lonenaustauscherharzes (Styroldivinylbenzolcopolymer mit Sulfonsäuregruppen, handelsüblich unter dem Namen ®Duolite C20) gerührt, bis der pH-Wert der wäßrigen Lösung 2,3 war. Anschließend wurde das lonenaustauscherharz abfiltriert und die wäßrige Lösung mit 1 molarer NaOH-Lösung auf einen pH-Wert von 4,7 eingestellt. Danach wurde das entstandene Gel noch 3 Stunden bei 85°C gealtert und anschließend das Wasser mit 3 1 Aceton gegen Aceton ausgetauscht. Danach wurde das acetonhaltige Gel mit einer Mischung aus Trimethylchlorsilan und Diphenylmethylchlorsilan silyliert (2 Gew.-% Trimethylchlorsilan pro Gramm nasses Gel und 2 Gew.-% Diphenylmethylchlorsilan pro Gramm nasses Gel). Die Trocknung des Gels erfolgte an Luft (3 Stunden bei 40°C, dann 2 Stunden bei 50°C und 12 Stunden bei 150°C).

Das so erhaltene, transparente Aerogel hatte eine Dichte von 0,15 g/cm³,eine spezifische Oberfläche nach BET von 480 m²/g und es war dauerhaft hydrophob.

Das so hergestellte, dauerhaft hydrophobe Aerogel wurde bei 450°C in einem leichten Luftstrom mittels einem Röhrenofen 20 Minuten pyrolysiert. Das erhaltene transparente Aerogel hatte eine Dichte von 0,17 g/cm³, eine spezifische Oberfläche nach BET von 450 m²/g, eine Wärmeleitfähigkeit λ von 22 mW/mK und war nur teilweise hydrophil.

Die Wärmeleitfähigkeit wurde mit einer Heizdrahtmethode (s. z.B. O. Nielsson, G. Rüschenpöhler, J. Groß, J. Fricke, High Temperatures - High Pressures, Vol. 21, 267-274 (1989)) gemessen.

## Patentansprüche

1. Verfahren zur Herstellung hydrophiler oder teilweise hydrophiler, anorganischer Aerogele, dadurch gekennzeichnet, daß man ein hydrophobes, anorganisches durch Silylierung modifiziertes Aerogel in Gegenwart von Sauerstoff bei Temperaturen im Bereich von 100 bis 1000°C pyrolysiert.

2. Verfahren gemäß Anspruch 1, dadurch gekennzeichnet, daß man als hydrophobes Aerogel ein unterkritsch getrocknetes Aerogel verwendet.

3. Verfahren gemäß Anspruch 1 oder 2, dadurch gekennzeichnet, daß man als hydrophobes Aerogel ein organisch modifiziertes Aerogel verwendet.

4. Verfahren gemäß mindestens einem der Ansprüche 1 bis 3, dadurch gekennzeichnet, daß man das Aerogel bei einer Temperatur im Bereich von 150 bis 800°C pyrolysiert.

5. Verfahren gemäß mindestens einem der Ansprüche 1 bis 4, dadurch gekennzeichnet, daß man die Pyrolyse an Luft durchführt.

6. Verfahren gemäß mindestens einem der Ansprüche 1 bis 4, dadurch gekennzeichnet, daß man die Pyrolyse in einem leichten Luftstrom durchführt.

## Claims

1. A method of producing hydrophilic or partially hydrophilic inorganic aerogels, characterised in that a hydrophobic inorganic aerogel modified by silylation is hydrolysed in the presence of oxygen at temperatures in the range from 100 to 1000°C.

2. A method according to claim 1, characterised in that a subcritically dried aerogel is used as the hydrophobic aerogel.

3. A method according to claim 1 or 2, characterised in that an organically modified acrogel is used as the hydrophobic aerogel.

4. A method according to at least one of claims 1 to 3, characterised in that the aerogel is pyrolysed at a temperature in the range from 150 to 800°C.

5. A method according to at least one of claims 1 to 4, characterised in that the pyrolysis is carried out in air.

6. A method according to at least one of claims 1 to 4, characterised in that pyrolysis is carried out in a light current of air.

## Revendications

1. Procédé de préparation d'aérogels inorganiques hydrophiles ou partiellement hydrophiles, caractérisé en ce qu'on pyrolyse un aérogel hydrophobe, inorganique modifié par silylation en présence d'oxygène à des températures dans l'intervalle de 100 à 1000°C.

2. Procédé selon la revendication 1, caractérisé en ce que l'on utilise comme aérogel hydrophobe un aérogel séché de manière sous-critique.

3. Procédé selon la revendication 1 ou 2, caractérisé en ce que l'on utilise comme aérogel hydrophobe un aérogel organique modifié.

4. Procédé selon au moins l'une des revendications 1 à 3, caractérisé en ce qu'on pyrolyse l'aérogel à une température dans l'intervalle de 150 à 800°C.

5. Procédé selon au moins l'une des revendications 1 à 4, caractérisé en ce qu'on effectue la pyrolyse à l'air.

6. Procédé selon au moins l'une des revendications 1 à 4, caractérisé en ce qu'on effectue la pyrolyse dans un léger courant d'air.
